(19) Europäisches Patentamt
European Patent Office
Office européen des brevets

(11) Veröffentlichungsnummer: **0 012 260**
B1

(12) # EUROPÄISCHE PATENTSCHRIFT

(45) Veröffentlichungstag der Patentschrift: **16.09.81**

(51) Int. Cl.³: **C 07 C 143/30,** C 07 C 139/00

(21) Anmeldenummer: **79104680.8**

(22) Anmeldetag: **24.11.79**

(54) **Neue sulfierungsprodukte des Naphthalins, Verfahren zu ihrer Herstellung und ihre Verwendung.**

(30) Priorität: **09.12.78 DE 2853337**

(43) Veröffentlichungstag der Anmeldung: **25.06.80 Patentblatt 80/13**

(45) Bekanntmachung des Hinweises auf die Patenterteilung: **16.09.81 Patentblatt 81/37**

(84) Benannte Vertragsstaaten: **BE CH DE FR GB IT NL SE**

(56) Entgegenhaltungen:
**DE-A-0 000 155**
**DE-A-2 730 157**
**FR-A-2 204 596**
**FR-A-2 386 519**
**FR-A-2 387 947**

(73) Patentinhaber: **BAYER AG, Zentralbereich Patente, Marken und Lizenzen, D-5090 Leverkusen 1, Bayerwerk (DE)**

(72) Erfinder: **Lindner, Otto, Dr., Straesschen Siefen 31, D-5060 Bergisch-Gladbach (DE)**
Erfinder: **Pelster, Heinrich, Dr., Auf dem Heidchen 23, D-5068 Odenthal (DE)**
Erfinder: **Steffan, Guido, Dr., Herzogenfeld 52, D-5068 Odenthal (DE)**
Erfinder: **Stüwe, Arnd, Dr., Erfurter Strasse 4, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 012 260 B1

ACTORUM AG.

# Neue Sulfierungsprodukte des Naphthalins, Verfahren zu ihrer Herstellung und ihre Verwendung

Die Erfindung betrifft neue Sulfierungsprodukte des Naphthalins, ein Verfahren zu ihrer Herstellung und ihre Verwendung als Zwischenprodukte z.B. für die Herstellung von Naphthalin-1,5-disulfonsäure.

Verfahren zur Sulfonierung von Naphthalin sind in grosser Zahl bekannt. So wird beispielsweise in den FR-A 2386519 und 2387947 die Herstellung von Naphthalin-1,3,6-trisulfonsäure durch Sulfonieren von Naphthalin mit Schwefelsäure und Oleum bei erhöhten Temperaturen beschrieben. In der DE-A 2730157 ist ein Verfahren beschrieben, wie man aus solchen, bei der Trisulfonierung von Naphthalin anfallenden Trisulfonierungsgemischen die überschüssige Schwefelsäure zurückgewinnen kann.

Die am Prioritätstag des vorliegenden Verfahrens noch nicht veröffentlichte Eu-A 0000155 betrifft ein Verfahren zur kontinuierlichen Herstellung von Naphthalin-1-sulfonsäure und -1,5-disulfonsäure, bei dem Naphthalin mit Schwefeltrioxid in Schwefelsäure bei niedrigen Temperaturen in einer Schneckenmaschine umgesetzt wird.

Die FR-A 2204596 betrifft ganz allgemein ein Verfahren zum Monosulfonieren mit Schwefeltrioxid sulfonierbarer aromatischer Verbindungen, u.a. Naphthalin. Gemäss diesem Verfahren wird die im Überschuss, bezogen auf Schwefeltrioxid, vorgelegte aromatische Verbindung mit einem inerten organischen Lösungsmittel verdünnt. In diese bei einer Temperatur unter 100°C bei normalem oder vermindertem Druck siedende Lösung wird gasförmiges Schwefeltrioxid mit einer bestimten Geschwindigkeit eingeleitet.

Auch andere Verfahren, Naphthalin mit $SO_3$ in inerten organischen Lösungsmitteln umzusetzen, sind bereits bekannt (s. Comptes rendus, Band 182, 1926, Seiten 856–857; Houben-Weyl «Methoden der Organischen Chemie», Band 9 (1955), Seite 502).

Gemäss Comptes rendus werden 3 Mol $SO_3$ nach und nach in eine Lösung von einem Mol Naphthalin in wasserfreiem Chloroform gegeben. Der sich bei der Umsetzung bildende Niederschlag wird als Additionsprodukt von 2 Mol $SO_3$ an ein Mol Naphthalin-1,5-disulfonsäure beschrieben. Dieses Additionsprodukt liefert bei seiner Hydrolyse Naphthalin-1,5-disulfonsäure in 50%iger Ausbeute.

Gemäss Houben-Weyl (loc. cit.) werden die Lösungen von 2,5 Mol $SO_3$ in Tetrachloräthan und 1 Mol Naphthalin in Tetrachloräthan bei 20°C gleichzeitig in vorgelegtes Tetrachloräthan getropft. Es entstehen Sulfonsäuren, die sich als farblose Körnchen abscheiden. Von diesen Sulfonsäuren wird ausgesagt, dass sie beim Lösen in Wasser und Aussalzen mit Natriumsulfat das Natriumsalz der Naphthalin-1,5-disulfonsäure in sehr reiner Form und in Ausbeuten von 74–75%, bezogen auf Naphthalin, ergeben. Die Nacharbeitung dieses Sulfierungsverfahrens ergibt jedoch, dass die Ausbeuten an Naphthalin-1,5-disulfonsäure tatsächlich nur 65–70%, bezogen auf eingesetztes Naphthalin, betragen.

Es wurde nun überaschenderweise gefunden, dass man bei der Sulfierung von Naphthalin mit $SO_3$ in inerten organischen Lösungsmitteln neue Naphthalinsulfierungsprodukte mit wesentlichen verbesserten Eigenschaften erhält, wenn man Naphthalin und $SO_3$ im Molverhältnis 1:3 bis 5, vorzugsweise 1:3 bis 3,6 einsetzt und Naphthalin und $SO_3$ in dem inerten Lösungsmittel bei Temperaturen von −40 bis +10°, vorzugsweise −20 bis 0°C, so zusammengibt, dass das Verhältnis von $SO_3$ zu Naphthalin während des Zusammengebens im Bereich von 3 bis 5, vorzugsweise 3 bis 3,6 Mol $SO_3$ je Mol Naphthalin liegt.

Die unter diesen Reaktionsbedingungen erhältlichen Naphthalinsulfierungsprodukte liefern bei ihrer Hydrolyse Naphthalin-1,5-disulfonsäure in hoher Reinheit und in einer Ausbeute bis zu 81%, bezogen auf eingesetztes Naphthalin.

Wie die analytische Untersuchung (IR-Spektren, Umsetzung mit Ammoniak und Aminen) ergeben hat, handelt es sich bei diesen neuen Naphthalinsulfierungsprodukten um oligomere Naphthalinsulfonsäureanhydride der nachstehend angegebenen Formel I.

Die Erfindung betrifft daher neue Sulfierungsprodukte des Naphthalins der Formel

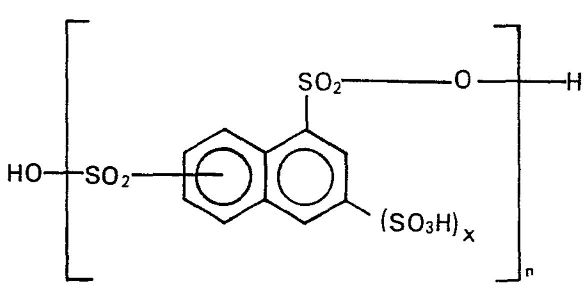 $\cdot (n-1)\ H_2SO_4 \cdot (n \cdot y) SO_3$ I

in der

n für eine ganze Zahl von 2 bis 10, vorzugsweise 2 bis 6, steht,

x 0 oder 1 bedeutet, x jedoch nur bei höchstens 10%, vorzugsweise höchstens 6% der insgesamt vorliegenden sulfierten Naphthalinreste 1 ist und

y 0 oder eine ganze oder gebrochene Zahl zwischen 0 und 2, vorzugsweise 0 und 1 bedeutet.

Die erfindungsgemässen Naphthalinsulfonsäureanhydride der Formel I halten gewisse Men-

gen an Schwefelsäure und $SO_3$ adsorptiv gebunden. Die Menge (in Mol) an adsorptiv gebundener Schwefelsäure entspricht der Anzahl der $-SO_2-O-SO_2$-Brücken. Bereits beim Behandeln der Naphthalinsulfonsäureanhydride mit Eiswasser wird diese Schwefelsäure abgespalten, ohne dass das Anhydrid in Lösung geht. Die Menge an adsorbtiv gebundenem $SO_3$ entspricht dem Überschuss an $SO_3$, der über die für die Sulfierung von Naphthalin und die Ausbildung der Anhydrid-Brücken erforderliche Menge von 3 Mol $SO_3$ je Mol Naphthalin hinaus eingesetzt wird.

Da die erfindungsgemässen Naphthalinsulfonsäureanhydride der Formel I überwiegend Einheiten enthalten, die sich von der Naphthalin-1,5-disulfonsäure ableiten und Naphthalin-1,5-disulfonsäure mit dem Trivialnamen Armstrongsäure bezeichnet wird, werden die erfindungsgemässen Naphthalinsulfonsäureanhydride nachstehend vereinfacht als Armstrongsäureanhydrid oder als AS-AN bezeichnet.

Das erfindungsgemässe AS-AN ist in reiner Form ein sehr stark hygroskopisches, rieselfähiges Pulver. Meist ist es farblos, gelegentlich auch schwach grau oder rosafarben. An feuchter Luft raucht es in Abhängigkeit vom Gehalt an adsorbtiv gebundenem $SO_3$ mehr oder weniger stark. In Eiswasser löst sich das Anhydrid nur langsam auf, schneller beim Erwärmen oder beim Zusatz von Alkalien. AS-AN ist bei Temperaturen über +10°C, insbesondere bei Raumtemperatur, mechanisch empfindlich. Unter Druck und Einfluss von Scherkräften verwandelt es sich bei Raumtemperatur von einem lockeren Pulver in eine klebrige, plastische Masse.

AS-AN ist thermisch bis etwa 40°C stabil. Durch Erwärmen auf Temperaturen bis 40°C ändert sich die Zusammensetzung der Hydrolyseprodukte praktisch nicht. Beim Erwärmen auf höhere Temperaturen finden offenbar Umlagerungen statt. Dies zeigt sich daran, dass bei der Verseifung eines auf höhere Temperaturen als 40°C erwärmten AS-AN die Menge an Naphthalintrisulfonsäure insbesondere auf Kosten von Naphthalin-1,6-disulfonsäure zunimmt.

Bei dem erfindungsgemässen AS-AN handelt es sich im wesentlichen um oligomere Anhydride der Naphthalin-1,5-disulfonsäure. Ausser Naphthalin-1,5-disulfonsäure sind noch untergeordnete Mengen (12–18 Mol-%) der anderen isomeren Naphthalin-disulfonsäuren vor allem Naphthalin-1,6-disulfonsäure und untergeordnete Mengen (2–8 Mol-%) Naphthalintrisulfonsäure an der Anhydrid-Bildung beteiligt.

Eine typische Zusammensetzung der erfindungsgemässen Naphthalinsulfonsäureanhydride ist z.B. die folgende:

Spalte A: Angaben in Gewichtsprozent; die das Anhydrid aufbauenden Naphthalinsulfonsäure-Einheiten sind als freie Sulfonsäuren und adsorptiv gebundene Schwefelsäure und $SO_3$ als Schwefeltrioxid angegeben.

Spalte B: Ausbeuten (in % der Theorie) an das Anhydrid aufbauenden Napthalinsulfonsäuren bezogen auf eingesetztes Naphthalin.

| | A | B |
|---|---|---|
| Naphthalin-1,3-disulfonsäure | 0,2 | 0,3 |
| Naphthalin-1,4-disulfonsäure | 0,02 | 0,03 |
| Naphthalin-1,5-disulfonsäure | 61,0 | 80,5 |
| Naphthalin-1,6-disulfonsäure | 6,4 | 8,4 |
| Naphthalin-1,7-disulfonsäure | 2,3 | 3,0 |
| Naphthalin-1,3,5-trisulfonsäure | 2,3 | 2,4 |
| Naphthalin-1,3,6-trisulfonsäure | 4,7 | 4,9 |
| Naphthalin-1,3,7-trisulfonsäure | 0,5 | 0,5 |
| Schwefeltrioxid | 22,5 | - |

Im IR-Spektrum weisen die erfindunsgemässen AS-AN die für Sulfonsäureanhydride charakteristische Bande bei etwa 1400 $cm^{-1}$ auf.

Bei der Umsetzung von AS-AN mit Ammoniak oder Aminen, z.B. Cyclohexylamin, werden Naphthalin-1,5-disulfonsäurediamide erhalten. Diese Amidbildung ist nur bei Vorliegen von Anhydridgruppen erklärlich. Würden nur freie Sulfonsäuregruppen vorliegen, wäre nur die Bildung von Naphthalinsulfonsäure-Ammoniumsalzen, nicht aber die Bildung von Naphthalin-Sulfonsäureamiden zu erwarten. Die Wasserunlöslichkeit des AS-AN bei gleichzeitigem Inlösunggehen von Schwefelsäure sowie die Mengen der bei der AS-AN-Aminolyse gebildeten Armstrongsäurediamide und Armstrongsäureamidsäuren sprechen gegen das Vorliegen gemischter Anhydride aus Napthalinsulfonsäuren und Schwefelsäure.

Die Erfindung betrifft ferner ein Verfahren zur Herstellung der erfindungsgemässen Naphthalinsulfierungsprodukte der Formel I. Das Verfahren ist dadurch gekennzeichnet, dass man Naphthalin und $SO_3$ im Molverhältnis 1:3 bis 5, vorzugsweise 1:3 bis 3,6 einsetzt und Naphthalin und $SO_3$ bei Temperaturen von -40 bis +10°C, vorzugsweise -20°C bis 0°C in einem inerten Lösungsmittel so zusammengibt, dass das Verhältnis von $SO_3$ zu Naphthalin während des Zusammengebens im Bereich 3 bis 5, vorzugsweise 3 bis 3,6 Mol $SO_3$ je Mol Naphthalin liegt.

Für das erfindungsgemässe Verfahren kann handelsübliches Naphthalin und handelsübliches, auf beliebige Weise hergestelltes $SO_3$ verwendet werden. Unter inerten Lösungsmitteln sind solche Lösungsmittel zu verstehen, die unter den Reaktionsbedingungen von $SO_3$ nicht oder praktisch nicht angegriffen werden und die zugleich ein ausreichendes Lösungsvermögen für Naphthalin und $SO_3$ haben. Besonders geeignet sind beispielsweise niedrig siedende, d.h. einen Siedepunkt unter 100°C aufweisende Chloralkane wie 1,2-Dichloräthan und 1,2-Dichlorpropan. Besonders bewährt hat sich Methylenchlorid. Brauchbar ist auch flüssiges Schwefeldioxid.

Die Menge an Lösungsmittel kann in weiten Grenzen schwanken. Im allgemeinen wird mindestens soviel Lösungsmittel eingesetzt, dass nach beendeter Reaktion eine noch gut rührbare Suspension vorliegt. Es hat sich bewährt, je Mol Naphthalin insgesamt 300 bis 3000 g Lösungsmittel einzusetzen. Bei Verwendung von Methylenchlorid hat es sich als vorteilhaft erwiesen, je Mol Naphthalin insgesamt 500 bis 1700 g Methylenchlorid zu verwenden.

Das Lösungsmittel kann auf verschiedene Weise in die Reaktion eingebracht werden. Beispielsweise kann man das gesamte einzusetzende Lösungsmittel im Reaktionsgefäss vorlegen, es auf Reaktionstemperatur bringen und dann $SO_3$ in flüssiger oder gasförmiger Form und Naphthalin in flüssiger Form eindosieren. Man kann auch nur einen Teil des einzusetzenden Lösungsmittels vorlegen und entweder Naphthalin in flüssiger Form und $SO_3$ gelöst im restlichen Lösungsmittel oder Naphthalin gelöst im restlichen Lösungsmittel und $SO_3$ in flüssiger oder gasförmiger Form zugeben. Man kann auch Naphthalin und $SO_3$, jeweils gelöst in einem Teil des Lösungsmittels, in das vorgelegte restliche Lösungsmittel zudosieren. Weiterhin kann man die gesamte einzusetzende Lösungsmittelmenge zum Lösen von Naphthalin und $SO_3$ verwenden und dann beide Lösungen getrennt, vorzugsweise gekühlt, in das Reaktionsgefäss eindosieren.

Besonders bewährt hat es sich, einen Teil des Lösungsmittels und/oder einen Teil des vorhergehenden Ansatzes vorzulegen und Naphthalin und $SO_3$ gelöst im Lösungsmittel zuzufügen.

Wesentlich für das Zusammengeben von $SO_3$ und Naphthalin ist, dass das Verhältnis von zugegebenem $SO_3$ zu zugegebenem Naphthalin stets im Bereich von 3 bis 5, vorzugsweise 3 bis 3,6 Mol liegt. Dies kann man erreichen, indem man $SO_3$ und Naphthalin, vorzugsweise jeweils in gelöster Form, im Verhältnis 3 bis 5 Mol $SO_3$ pro Mol Naphthalin gleichmässig zusammengibt (Simultan-Dosierung). Man kann auch abwechselnd kleine Portionen von $SO_3$ und Naphthalin zudosieren. Es hat sich bewährt, vor Beginn des Zudosierens von Naphthalin und $SO_3$ eine kleine Menge $SO_3$ vorzulegen.

Die Reaktion von $SO_3$ mit Naphthalin verläuft unter den erfindungsgemässen Bedingungen sehr schnell und ist im allgemeinen wenige Minuten nach der Beendigung des Zudosierens der Komponenten beendet. Die Reaktion lässt sich deshalb auch in einfacher Weise kontinuierlich durchführen. Die Geschwindigkeit des Zudosierens der Komponenten $SO_3$ und Naphthalin kann beliebig gewählt werden, wenn für eine ausreichende Abfuhr der Reaktionswärme (etwa 500 kcal pro kg Naphthalin) gesorgt wird. Die Abführung der Reaktionswärme kann beispielsweise durch Wandkühlung erfolgen. Bevorzugt ist jedoch die Abführung der Reaktionswärme durch Siedekühlung gegebenenfalls im Vakuum. Mit dieser Arbeitsweise können schnell grössere Wärmemengen abgeführt werden und damit kürzere Dosierzeiten und höhere Raum/Zeit-Ausbeuten erreicht werden. Die Dosierzeit kann bei Anwendung der Siedekühlung beispielsweise 10 bis 30 Minuten betragen. Längere Dosierzeiten sind möglich, erbringen jedoch im allgemeinen keine Vorteile. Vorzugsweise wird das Reaktionsgemisch nach beendeter Dosierung kurze Zeit, beispielsweise 2 bis 60 Minuten nachgerührt.

Bei der Herstellung des erfindungsgemässen AS-AN hat es sich als vorteilhaft erwiesen, der Reaktionsmischung unmittelbar nach Beendigung des Zusammengebens von Naphthalin und $SO_3$ eine geringe Menge Wasser, beispielsweise bis zu 10 g Wasser, bezogen auf 1 Mol Naphthalin, zuzusetzen. Durch diesen Wasserzusatz wird ein AS-AN mit einem besonders niedrigen Gehalt an trisulfierten Naphthalineinheiten erhalten.

Das erfindungsgemässe Verfahren kann unter Normaldruck, erhöhtem Druck oder erniedrigtem Druck durchgeführt werden. Es ist lediglich darauf zu achten, dass stets eine hinreichende Menge an in flüssiger Form vorliegendem Lösungsmittel vorhanden ist, d.h. dass im Falle des Siedens, z.B. bei Wärmeabfuhr durch Siedekühlung, eine ausreichende Menge Lösungsmittel flüssig verbleibt oder durch entsprechendes Kühlen wieder verflüssigt wird. Bei Wärmeabfuhr durch Wandkühlung arbeitet man vorzugsweise bei Normaldruck. Bei Wärmeabfuhr durch Siedekühlung stellt man den Druck vorteilhaft so ein, dass das verwendete Lösungsmittel bei der gewünschten Reaktionstemperatur siedet.

Aus diesem Grund ist der Einsatz solcher Lösungsmittel bevorzugt, die bei Normaldruck einen relativ niedrigen Siedepunkt aufweisen. Methylenchlorid ist als Lösungsmittel u.a. auch deshalb besonders bevorzugt, weil bei seiner Verwendung und bei der Wärmeabfuhr durch Siedekühlung der anzuwendende Druck nicht zu niedrig liegt. Bei Verwendung von Methylenchlorid bei Reaktionstemperaturen von -5 bis -10 °C und bei Ableitung der Wärme durch Siedekühlung kann man beispielsweise bei Drucken im Bereich von 80 bis 150 mbar arbeiten.

Nach Beendigung der Reaktion liegt eine Suspension des Naphthalinsulfonsäureanhydrids (AS-AN) im jeweils verwendeten Lösungsmittel vor. Aus dieser Suspension kann man das reine, trockene AS-AN gewinnen, indem man die festen Anteile der Suspension abtrennt, z.B. durch Filtrieren, und anschliessend trocknet. Da die flüssige Phase praktisch keine Ausgangsverbindungen mehr enthält, kann man die Suspension auch ohne Filtration direkt zur Trockne einengen.

Das von der Suspension abgetrennte, bzw. beim Einengen der Suspension anfallende Naphthalinsulfonsäureanhydrid kann durch anschliessendes Trocknen von Resten anhaftenden Lösungsmittels befreit werden. Die Trocknung wird vorzugsweise unter erniedrigtem Druck und bei Temperaturen unter 50 °C ausgeführt. Da sich AS-AN bei Temperaturen über +10 °C durch Pressen und unter dem Einfluss von Scherkräften in eine klebrige plastische Masse verwandelt, werden vorzugsweise bei Trocknungstemperaturen über +10 °C Trockner verwendet, die nur geringe mechanische Kräfte auf das Produkt ausüben, z.B. Taumeltrockner. Bei Trocknungstemperaturen unter +10 °C, insbesondere unter 0 °C können ohne Nachteile auch Trockner verwendet werden, die das Produkt mechanisch stärker beanspruchen, z.B.Trockner mit rotierenden Einbauten.

Für die Weiterverarbeitung des erfindungsgemässen AS-AN ist es vielfach nicht erforderlich, dieses aus der Suspension als trockenen Feststoff zu gewinnen und/oder es von den Resten

des anhaftenden Lösungsmittels zu befreien. Wenn das Lösungsmittel bei der weiteren Verarbeitung des Armstrongsäureanhydrids nicht stört, kann das AS-AN sowohl in Form der bei der Umsetzung anfallenden Suspension als auch als lösungsmittelfeuchtes Produkt weiterverarbeitet werden.

Das erfindungsgemässe AS-AN kann beispielsweise zur Herstellung von Armstrongsäure verwendet werden. Bei dieser Herstellung kann man so verfahren, dass man das Armstrongsäureanhydrid mit Wasser bei erhöhter Temperatur hydrolysiert und anschliessend durch Temperaturerniedrigung das Armstrongsäuretetrahydrat ausfällt, abtrennt und gegebenenfalls mit Schwefelsäure wäscht. Beispielsweise kann die Hydrolyse bei 60 bis 100 °C und das Ausfällen des Tetrahydrates bei 5 bis 30 °C durchgeführt werden. Auf diese Weise wird Armstrongsäure in Ausbeuten bis zu 80% der Theorie, bezogen auf zur AS-AN-Herstellung eingesetztes Naphthalin, erhalten.

Armstrongsäure kann auf verschiedene Weise weiterverwendet werden, beispielsweise als Beizmittel und zur Herstellung von Azurinsäure und Azurol, die ihrerseits wichtige Farbstoffvorprodukte sind. Aus Azurol kann durch Umsetzung mit Ammoniak 1,5-Diaminonaphthalin und aus diesem durch Umsetzung mit Phosgen 1,5-Naphthalindiisocyanat hergestellt werden, das für die Herstellung von Polyurethanen verwendet werden kann. Alle diese Verwendungen von Armstrongsäure sind bekannt (s. z.B. Ullmann's Enzyklopädie der Technischen Chemie, 3. Auflage (1960), Band 12, Seiten 595, 605, 610 und 624; Ullmann a.a.O., Band 9, Seiten 1 ff; Ullmann a.a.O., 4. Auflage (1974), Band 8, Seiten 244 ff; Ullmann a.a.O., Band 13, Seiten 347 ff; FIAT Final Report Nr. 1313 I, Seiten 285 ff).

Beispiel 1

a) Herstellung von Armstrongsäureanhydrid

In einem Kessel aus nichtrostendem Stahl von 250 l Inhalt werden 50 kg Methylenchlorid vorgelegt. Durch Anlegen von Vakuum (etwa 120 mbar) wird eine Kesselinnentemperatur von -5 bis -10 °C eingestellt. Im Verlaufe von 2 Stunden werden auf -10 °C gekühlte Lösungen von 32 kg (400 Mol) Schwefeltrioxid in 130 kg Methylenchlorid und von 16 kg (125 Mol) Naphthalin in 80 kg Methylenchlorid gleichzeitig so in den Kessel eindosiert, dass innerhalb von 5 Minuten jeweils 8,5 kg der Schwefeltrioxid-Lösung und 5 kg der Naphthalin-Lösung einlaufen.

Durch die Reaktionswärme verdampfen bei einer Kesselinnentemperatur von etwa -7 °C etwa 100 kg Methylenchlorid. Das verdampfende Methylenchlorid wird kondensiert und in einer gekühlten Vorlage aufgefangen.

Das AS-AN fällt als farbloses Pulver aus dem Reaktionsgemisch aus. Die entstandene Suspension wird in einem emaillierten Taumeltrockner im Vakuum bei Temperaturen von 0 bis 10 °C zur Trockne eingedampft.

Es werden 47,4 kg AS-AN in Form eines farblosen, an feuchter Luft schwach rauchenden Pulvers erhalten.

Das Pulver weist im IR-Spektrum neben den für freie aromatische Sulfonsäuren charakteristischen Banden die für Sulfonsäureanhydride charakteristische Bande bei 1400 $cm^{-1}$ auf.

Die Zusammensetzung des Anhydrides wurde durch Hochdruck-Flüssigkeitschromatographie einer verdünnten wässrigen, mit Natronlauge neutralisierten Lösung und durch Bestimmung des Schwefelsäure-Gehaltes der wässrigen Lösung bestimmt. Aus den Analysendaten ergab sich folgende Zusammensetzung:

| | |
|---|---|
| Naphthalin-1,3-disulfonsäure | 0,2 Gew.-% |
| Naphthalin-1,4-disulfonsäure | 0,02 Gew.-% |
| Naphthalin-1,5-disulfonsäure | 61,0 Gew.-% |
| Naphthalin-1,6-disulfonsäure | 6,4 Gew.-% |
| Naphthalin-1,7-disulfonsäure | 2,3 Gew.-% |
| Naphthalin-1,3,5-trisulfonsäure | 2,3 Gew.-% |
| Naphthalin-1,3,6-trisulfonsäure | 4,7 Gew.-% |
| Naphthalin-1,3,7-trisulfonsäure | 0,5 Gew.-% |
| $SO_3$ | 22,5 Gew.-% |

(die Zahlenangabe für $SO_3$ umfasst Schwefeltrioxid und den $SO_3$-Gehalt der adsorbtiv gebundenen Schwefelsäure).

b) Verseifung des Armstrongsäureanhydrids zur Armstrongsäure

Die gemäss a) erhaltene Suspension des AS-AN in Methylenchlorid, enthaltend 47,4 kg Armstrongsäureanhydrid, wurde in einem 160-l-Stahl-Email-Kessel in 33 kg vorgelegtes Wasser von 60 °C so eindosiert, dass die Temperatur im Kessel auf 70 bis 75 °C gehalten werden konnte. Danach wurde bei 70 bis 80 °C Vakuum angelegt und der Druck allmählich auf 100 mbar gesenkt, bis das Methylenchlorid vollständig abdestilliert war. Unter langsamem Rühren wurde dann im Verlauf von 6 Stunden auf 15 °C abgekühlt. Aus der entstehenden dickflüssigen Suspension wurde das Armstrongsäuretetrahydrat mittels einer Schälschleuder isoliert.

Nach dreimaligem Waschen mit je 5 kg 48%iger wässriger Schwefelsäure wurde das Armstrongsäuretetrahydrat trockengeschleudert. Es wurden 43 kg schwefelsäurefeuchtes Armstrongsäuretetrahydrat (Gehalt an Schwefelsäure etwa 7,6 Gew.-%) erhalten.

Das Rohprodukt enthält neben 66,8 Gew.-% Naphthalin-1,5-disulfonsäure noch 0,33 Gew.-% isomere Naphthalindisulfonsäuren (vornehmlich Naphthalin-1,6-disulfonsäure und etwa 0,1 Gew.-% Naphthalintrisulfonsäuren. Die Ausbeute an reinem Naphthalin-1,5-disulfonsäuretetrahydrat (Armstrongsäuretetrahydrat) beträgt 79,8% der Theorie, bezogen auf eingesetztes Naphthalin.

Beispiele 2 und 3

Es wurde wie in Beispiel 1 beschrieben verfahren, nur wurde die Sulfierung statt bei -7 °C

a) bei -20 °C und

b) bei 0 °C

durchgeführt.

Die auf diese Weise erhaltenen AS-AN ergaben bei ihrer Hydrolyse Armstrongsäure-Tetrahydrat im Falle

a) in einer Ausbeute von 81%, im Falle
b) in einer Ausbeute von 78,6%,
bezogen auf eingesetztes Naphthalin.

Beispiele 4 bis 6

In 1000 ml trockenes, auf -10 °C gekühltes Methylenchlorid wurden unter gutem Rühren gleichzeitig 256 g (2 Mol) geschmolzenes Naphthalin von 100 °C und

a) 480 g (6 Mol) gasförmiges $SO_3$
b) 512 g (6,4 Mol) gasförmiges $SO_3$ und
c) 560 g (7 Mol) gasförmiges $SO_3$

eingeleitet. Das Einleiten des $SO_3$ dauerte etwa eine Stunde. Die Temperatur der Reaktionsmischung wurde durch Aussenkühlung bei -7 bis -11 °C gehalten. Die Reaktionsmischung wurde 15 Minuten bei 0 °C nachgerührt. Anschliessend wurde der ausgeschiedene Niederschlag schnell über eine grosse Glasfritte abgesaugt und im Vakuum bei 20 °C getrocknet. Das AS-AN fiel bei allen drei Versuchen nach dem Trocknen in Form eines feinen Pulvers an. Die Ausbeute an AS-AN betrug im Falle

a) 733 g
b) 761 g
c) 809 g

Die Hydrolyse der Armstrongsäureanhydride lieferte Roh-Naphthalin-1,5-disulfonsäuren, die noch 0,3-0,6 Gew.-% isomere Naphthalin-disulfonsäuren und 0,0-0,2 Gew.-% Naphthalintrisulfonsäuren (jeweils bezogen auf reine 1,5-Disulfonsäure) enthielten.

Die Ausbeute an reinem Armstrongsäuretetrahydrat betrug im Falle

a) 78,4% d.Th.
b) 79,6% d.Th.
c) 79,1% d.Th., jeweils bezogen auf eingesetztes Naphthalin.

Beispiel 7

In 1000 ml trockenes 1,2-Dichloräthan werden unter gutem Rühren bei -25 °C unter Aussenkühlung im Verlauf von 2 Stunden gleichzeitig 256 g (2 Mol) geschmolzenes Naphthalin von 100 °C und 536 g (6,7 Mol) flüssiges Schwefeltrioxid von 40 °C so zugetropft, dass beide Verbindungen zum gleichen Zeitpunkt vollständig eindosiert waren und das Dosierverhältnis während der gesamten Dosierzeit gleich blieb. Es wurde 1 Stunde bei -10 °C und 30 Minuten bei 0 °C nachgerührt. Dann wurde der pulverförmige Niederschlag abgesaugt.

Das auf diese Weise erhaltene, lösungsmittelfeuchte pulverförmige AS-AN wurde in 755 ml Wasser gelöst und die wässrige Lösung von dem sich abscheidenen 1,2-Dichloräthan abgetrennt. Die wässrige Lösung wurde auf 70 °C erwärmt, bei 70 bis 80 °C mit 290 g 100%iger Schwefelsäure versetzt und dann unter Rühren auf +10 °C abgekühlt. Das beim Abkühlen auskristallisierende Armstrongsäuretetrahydrat wurde über eine Fritte abgesaugt und dreimal mit je 50 ml auf +10 °C gekühlter 50%iger Schwefelsäure gewaschen. Es wurden 895 g schwefelsäurefeuchtes Armstrongsäuretetrahydrat erhalten.

Gehalt des schwefelsäurefeuchten Produktes (gemäss Hochdruckflüssigkeitschromatographie und Elementaranalyse) an Armstrongsäure: 455 g (= 79% der Theorie bezogen auf eingesetztes Naphthalin).

Wurden statt 1000 ml trockenem 1,2-Dichloräthan die gleiche Menge trockenes 1,2-Dichlorpropan verwendet, so betrug die Ausbeute an Armstrongsäure 78,6% bezogen auf eingesetztes Naphthalin.

Beispiel 8

Die Sulfierung des Naphthalins mit Schwefeltrioxid wurde wie in Beispiel 1a) beschrieben, durchgeführt. Nach beendeter Zugabe von Schwefeltrioxid und Naphthalin wurde die Reaktionsmischung noch 10 Minuten bei -7 bis -8 °C nachgerührt. Anschliessend wurden im Verlauf von 15 Minuten unter schnellem Rühren bei -7 bis -5 °C 0,9 kg Wasser in die erhaltene Armstrongsäureanhydrid-Suspension in fein verteilter Form eingeleitet (eingedüst). Die mit Wasser versetzte Reaktionsmischung wurde noch 30 Minuten nachgerührt. Dann wurde das ausgefallene Reaktionsprodukt über eine Nutsche abfiltriert und nach kurzem Nachblasen mit Stickstoff in einem Vakuumtrockenschrank bei 20 bis 25 °C und einem Druck von 400 bis 25 mbar getrocknet.

Es wurden 48,7 kg AS-AN in Form eines schwach grauen amorphen Pulvers erhalten.

Die Hydrolyse des Armstrongsäureanhydrids lieferte ein Roh-Armstrongsäure-Tetrahydrat, das 0,2 Gew.-% isomere Naphthalin-disulfonsäuren und 0,05 Gew.-% Naphthalin-1,3,5-trisulfonsäure enthielt. Die Ausbeute an reinem Armstrongsäuretetrahydrat betrug 80,5% der Theorie bezogen auf eingesetztes Naphthalin.

**Patentansprüche**

1. Sulfierungsprodukte des Naphthalins der Formel

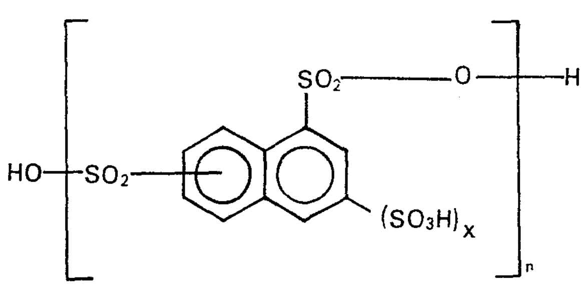

$\cdot (n-1)\, H_2SO_4 \cdot (n \cdot y) SO_3$

in der

n für eine ganze Zahl von 2 bis 10 steht,

x 0 oder 1 bedeutet, x jedoch nur bei höchstens 10% der insgesamt vorliegenden sulfierten Naphthalinreste 1 ist und

y 0 oder eine ganze Zahl oder gebrochene Zahl zwischen 0 und 2 bedeutet.

2. Sulfierungsprodukte des Naphthalins gemäss Anspruch 1, in denen

n für eine ganze Zahl von 2 bis 6 steht,

x 0 oder 1 bedeutet, x jedoch nur bei höchstens 6% der insgesamt vorliegenden Naphthalinreste 1 ist und

y 0 oder 1 oder eine gebrochene Zahl zwischen 0 und 1 bedeutet.

3. Verfahren zur Herstellung von Sulfierungsprodukten des Naphthalins gemäss Ansprüchen 1 und 2 durch Umsetzung von Naphthalin mit $SO_3$ in Gegenwart eines inerten Lösungsmittels, dadurch gekennzeichnet, dass man Naphthalin und $SO_3$ in dem inerten Lösungsmittel bei Temperaturen von -40 bis +10°C so zusammengibt, dass das Verhältnis von $SO_3$ zu Naphthalin während des Zusammengebens im Bereich von 3 bis 5 Mol $SO_3$ je Mol Naphthalin liegt.

4. Verfahren zur Herstellung von Sulfierungsprodukten gemäss Anspruch 3, dadurch gekennzeichnet, dass das Verhältnis von $SO_3$ zu Naphthalin während des Zusammengebens im Bereich von 3 bis 3,6 Mol $SO_3$ je Mol Naphthalin liegt.

5. Verfahren nach Anspruch 3 und 4, dadurch gekennzeichnet, dass man als Lösungsmittel Chloralkane oder flüssiges Schwefeldioxid einsetzt.

6. Verfahren nach Anspruch 3 und 4, dadurch gekennzeichnet, dass man als Lösungsmittel Methylenchlorid einsetzt.

7. Verfahren nach Anspruch 3 bis 6, dadurch gekennzeichnet, dass man die Umsetzung bei -20 bis 0°C durchführt.

8. Verfahren nach Anspruch 3 bis 7, dadurch gekennzeichnet, dass man nach beendeter Zugabe von Naphthalin und $SO_3$ dem Reaktionsgemisch bis zu 10 g Wasser pro Mol Naphthalin zusetzt.

9. Verwendung des Sulfierproduktes des Naphthalins gemäss Anspruch 1 zur Herstellung von Naphthalin-1,5-disulfonsäure.

**Claims**

1. Sulphonation products of naphthalene, of the formula

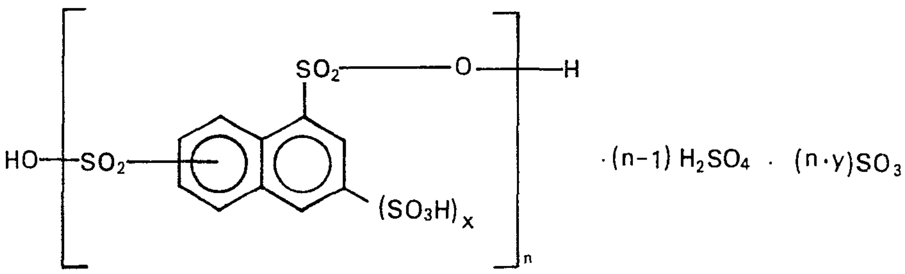

in which

n represents an integer from 2 to 10,

x denotes 0 or 1, but is 1 in the case of only at the most 10% of the total sulphonated naphthalene radicals present and

y denotes 0 or an integer or fraction between 0 and 2.

2. Sulphonation products of naphthalene according to Claim 1, in which

n represents an integer from 2 to 6,

x denotes 0 or 1, but is 1 in the case of only at the most 6% of the total naphthalene radicals present and

y denotes 0 or 1 or a fraction between 0 and 1.

3. A process for the preparation of sulphonation products of naphthalene according to Claims 1 and 2 by reacting naphthalene with $SO_3$ in the presence of an inert solvent, characterised in that naphthalene and $SO_3$ are brought together in the inert solvent at temperatures of -40 to +10°C, in such a way that the ratio of $SO_3$ to naphthalene during the mixing is in the range of 3 to 5 mol of $SO_3$ per mol of naphthalene.

4. A process for the preparation of sulphonation products according to Claim 3, characterised in that the ratio of $SO_3$ to naphthalene during the mixing is in the range of 3 to 3.6 mol of $SO_3$ per mol of naphthalene.

5. A process according to Claims 3 and 4, characterised in that chloroalkanes or liquid sulphur dioxide are used as the solvent.

6. A process according to Claims 3 and 4, characterised in that methylene chloride is used as the solvent.

7. A process according to Claims 3 to 6, characterised in that the reaction is conducted at -20 to 0°C.

8. A process according to Claims 3 to 7, characterised in that, after the addition of naphthalene and $SO_3$ has ended up to 10 g of water are added to the reaction mixture per mol of naphthalene.

9. The use of the sulphonation product of naphthalene according to Claim 1 for the preparation of naphthalene-1,5-disulphonic acid.

**Revendications**

1. Produits de sulfonation du naphtalène répondant à la formule

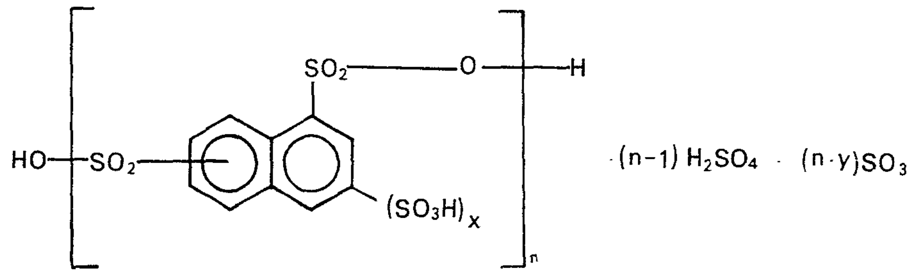

dans laquelle:
n est un nombre entier de 2 à 10,
x est égal à 0 ou 1, mais n'est égal à 1 que pour 10% au maximum des restes de naphtalène sulfonés totaux présents, et
y est égal à 0 ou représente un nombre entier ou fractionnaire de 0 à 2.

2. Produits de sulfonation du naphtalène selon la revendication 1, dans lesquels n est un nombre entier de 2 à 6, x est égal à 0 ou 1, mais n'est égal à 1 que pour 6% au maximum des restes de naphtalène totaux présents, et y est égal à 0 ou 1 ou représente un nombre fractionnaire entre 0 et 1.

3. Procédé de préparation de produits de sulfonation du naphtalène selon les revendications 1 et 2, par réaction du naphtalène avec $SO_3$, en présence d'un solvant inerte, caractérisé en ce que l'on mélange le naphtalène et $SO_3$ dans le solvant inerte à des températures de -40 à +10°C dans des conditions telles que, durant le mélange, le rapport de $SO_3$ au naphtalène se situe dans l'intervalle de 3 à 5 moles de $SO_3$ par mole de naphtalène.

4. Procédé de préparation de produits de sulfonation selon la revendication 3, caractérisé en ce que le rapport de $SO_3$ au naphtalène au cours du mélange se situe dans l'intervalle de 3 à 3,6 moles de $SO_3$ par mole de naphtalène.

5. Procédé selon les revendications 3 et 4, caractérisé en ce que l'on utilise comme solvant un chloralcane ou l'anhydride sulfureux liquéfié.

6. Procédé selon les revendications 3 et 4, caractérisé en ce que l'on utilise comme solvant le chlorure de méthylène.

7. Procédé selon les revendications 3 à 6, caractérisé en ce que l'on effectue la réaction entre -20 et 0°C.

8. Procédé selon les revendications 3 à 7, caractérisé en ce que, lorsque l'addition du naphtalène et de $SO_3$ est terminée, on ajoute au mélange de réaction une quantité d'eau allant jusqu'à 10 g par mole de naphtalène.

9. Utilisation du produit de sulfonation du naphtalène selon la revendication 1, pour la préparation de l'acide naphtalène-1,5-disulfonique.